# EUROPÄISCHE PATENTANMELDUNG

(11) **EP 3 241 819 A1**
(43) Veröffentlichungstag der Anmeldung: **08.11.2017**
(21) Anmeldenummer: 16167971.7
(22) Anmeldetag: 02.05.2016
(51) Int. Cl.: C07C 2/82, C07C 4/04, C07C 7/00, C07C 7/04, C07C 11/04, C07C 9/06, F25J 3/02

(54) **VERFAHREN ZUR DEMETHANISIERUNG UND DEMETHANIZER**

(71) Anmelder: Linde Aktiengesellschaft, 80331 München (DE)
(72) Erfinder: Pham Duc, Tuat, 82377 Penzberg (DE)
(74) Vertreter: m patent group

(57) **Zusammenfassung**

Es wird ein Verfahren zur Demethanisierung eines Kohlenwasserstoffe mit zwei Kohlenstoffatomen, Wasserstoff und Methan enthaltenden Gasgemischs, bei dem unter Verwendung des Gasgemischs eine leichte Fraktion, die zumindest den überwiegenden Anteil der Kohlenwasserstoffe mit zwei Kohlenstoffatomen aus dem Gasgemisch enthält, bereitgestellt wird, vorgeschlagen. Es ist vorgesehen, dass das Gasgemisch auf einem ersten Druckniveau einem oder mehreren Abkühlschritten unterworfen wird, dass nach dem oder den Abkühlschritten ein oder jeweils ein Kondensat aus dem Gasgemisch abgeschieden wird, dass das oder die Kondensate zusammen mit einem nach dem oder den Abkühlschritten gasförmig verbliebenen Anteil des Gasgemischs auf einem zweiten Druckniveau unterhalb des ersten Druckniveaus einer fraktionierten Destillation unterworfen werden, und dass der nach dem oder den Abkühlschritten gasförmig verbliebene Anteil des Gasgemischs dabei arbeitsleistend auf das zweite Druckniveau entspannt wird, bevor er der fraktionierten Destillation unterworfen wird. Ein entsprechender Demethanizer (100) ist ebenfalls Gegenstand der vorliegenden Erfindung.

## Beschreibung

Die vorliegende Erfindung betrifft ein Verfahren zur Demethanisierung eines unter Verwendung eines Verfahrens zur oxidativen Kopplung von Methan gebildeten Gasgemischs sowie einen entsprechenden Demethanizer gemäß den Oberbegriffen der jeweiligen unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet.

Die Reaktionsbedingungen bei der oxidativen Kopplung von Methan umfassen klassischerweise eine Temperatur von 500 bis 900 °C und einen Druck von 1 bis 10 bar sowie hohe Raumgeschwindigkeiten. Jüngere Entwicklungen gehen insbesondere auch in Richtung der Verwendung tieferer Temperaturen. Die Reaktion kann homogenund heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden; der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen beträchtliche Mengen nicht umgesetzten Methans sowie Wasser, Kohlenmonoxid und Kohlendioxid. Ein derartiges Gasgemisch wird nachfolgend auch als "Produktgemisch" der oxidativen Kopplung von Methan bezeichnet, obwohl es nicht nur die gewünschten Produkte, sondern auch nicht umgesetzte Edukte und Nebenprodukte enthält.

Bei der oxidativen Kopplung von Methan können auch Reaktoren eingesetzt werden, in denen einer katalytischen Zone eine nichtkatalytische Zone nachgeschaltet ist. Das in der katalytischen Zone gebildete Produktgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges Dampfspalten in einer der katalytischen Zone nachgeschalteten nichtkatalytischen Zone eines oder mehrerer Reaktoren wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2009, DOI 10.1002/14356007.a10_045.pub3, oder Falqi, F.: "The Miracle of Petrochemicals. Olefins Industry: An In-Depth Look at Steam-Crackers", Universal-Publishers, Boca Raton 2009, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Ist nachfolgend davon die Rede ist, dass "unter Verwendung eines Verfahrens zur oxidativen Kopplung von Methan" ein Produkt- oder Gasgemisch gebildet wird oder wurde, soll hierunter verstanden werden, dass hierbei ein Verfahren beteiligt ist oder war, das die zuvor erläuterten Reaktionen umfasst, insbesondere die Bildung von Methylradikalen, deren Kopplung zu Ethan und die anschließende Oxydehydrierung. Zusätzlich können jedoch weitere Verfahren bzw. Verfahrensschritte umfasst sein, insbesondere das erläuterte postkatalytische Dampfspalten. Diesem oder diesen weiteren Verfahren oder Verfahrensschritten können weitere Einsätze zugeführt werden, die zumindest teilweise zu Produkten umgesetzt werden, welche sich in dem Produkt- oder Gasgemisch wiederfinden. Mit anderen Worten muss bei der Bildung des Produkt- oder Gasgemischs, die "unter Verwendung" des Verfahrens zur oxidativen Kopplung von Methan erfolgt, im Rahmen der vorliegenden Erfindung nicht ausschließlich die oxidative Kopplung von Methan beteiligt sein. Das Gasgemisch kann auch unter Verwendung geeigneter Aufbereitungs- und/oder Fraktionierungsschritte aus einem entsprechenden Produktgemisch gebildet worden sein, wie auch nachfolgend noch erläutert. Es ist aber auch dann noch "unter Verwendung eines Verfahrens zur oxidativen Kopplung von Methan" gebildet worden.

Produkt- bzw. Gasgemische, die unter Verwendung eines Verfahrens zur oxidativen Kopplung von Methan gebildet wurden, enthalten aus den oben erläuterten Gründen insbesondere große Mengen an Methan, das vorteilhafterweise abgetrennt und in die oxidative Kopplung zurückgeführt oder anderweitig genutzt werden sollte. Die trenntechnische Bearbeitung eines entsprechenden Produkt- bzw. Gasgemischs stellt sich dabei aufgrund des hohen Methananteils bei dem vergleichsweise geringem Gehalt an Zielprodukten als ausgesprochen aufwendig dar.

Die vorliegende Erfindung will daher verbesserte Möglichkeiten zur Bearbeitung entsprechender Produkt- bzw. Gasgemische aufzeigen.

### Offenbarung der Erfindung

Die vorliegende Erfindung schlägt ein Verfahren zur Demethanisierung eines unter Verwendung eines Verfahrens zur oxidativen Kopplung von Methan gebildeten Gasgemischs sowie einen Demethanizer mit den Merkmalen der jeweiligen unabhängigen Patentansprüche vor. Ausgestaltungen sind jeweils Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Die vorliegende Anmeldung verwendet zur Charakterisierung von Drücken und Temperaturen die Begriffe "Druckniveau" und "Temperaturniveau", wodurch zum Ausdruck gebracht werden soll, dass Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere umfassen beispielsweise Druckniveaus auch unterschiedliche Drücke, die sich durch unvermeidliche Druckverluste ergeben. Entsprechendes gilt für Temperaturniveaus. Bei hier in bar angegebenen Druckniveaus handelt es sich um Absolutdrücke.

In Verfahren und Anlagen der eingangs erläuterten Art können zur Verdichtung mehrstufige Turboverdichter zum Einsatz kommen. Der mechanische Aufbau von Turboverdichtern ist dem Fachmann grundsätzlich bekannt. In einem Turboverdichter erfolgt die Verdichtung des zu verdichtenden Mediums mittels Turbinenschaufeln, die auf einem Turbinenrad oder direkt auf einer Welle angeordnet sind. Ein Turboverdichter bildet dabei eine bauliche Einheit, die jedoch bei einem mehrstufigen Verdichter mehrere Verdichterstufen aufweisen kann. Eine Verdichterstufe umfasst dabei in der Regel ein Turbinenrad oder eine entsprechende Anordnung von Turbinenschaufeln. Alle dieser Verdichterstufen können von einer gemeinsamen Welle angetrieben werden. Es kann jedoch auch vorgesehen sein, die Verdichterstufen gruppenweise mit unterschiedlichen Wellen anzutreiben, wobei die Wellen auch über Getriebe miteinander verbunden sein können. Ein Gebläse ist eine Form eines Verdichters, der nicht als Turboverdichter ausgebildet sein muss, und in dem eine Verdichtung in vergleichsweise geringem Umfang erfolgt. Ein Gebläse wird typischerweise eingesetzt, um einen Leitungswiderstand oder Höhenunterschied zu überwinden und damit den Transport eines gasförmigen Fluids sicherzustellen, nicht jedoch, dieses im eigentlichen Sinne zu verdichten.

Eine Entspannungsmaschine, die im Rahmen der vorliegenden Erfindung zur arbeitsleistenden Entspannung eines Gasgemischs eingesetzt werden kann, ist insbesondere als Turboexpander ausgebildet. Ein Turboexpander zeigt grundsätzlich bauliche Ähnlichkeiten zu einem Turboverdichter. Auch bei einem Turboexpander sind Turbinenschaufeln vorgesehen, die auf einem Turbinenrad oder direkt auf einer Welle angeordnet sein können. Durch die Entspannung wird ein Drehmoment in die Welle eingeleitet, das auch mechanisch umgesetzt werden kann, beispielsweise in einer Bremse oder einem Generator.

Ein Wärmetauscher dient zur indirekten Übertragung von Wärme zwischen zumindest zwei z.B. im Gegenstrom zueinander geführten Fluidströmen. Ein Wärmetauscher zu Einsatz im Rahmen der vorliegenden Erfindung kann aus einem einzelnen oder mehreren parallel und/oder seriell verbundenen Wärmetauscherabschnitten gebildet sein, z.B. aus einem oder mehreren Plattenwärmetauscherblöcken.

Zur Auslegung und spezifischen Ausgestaltung von Destillationssäulen, wie sie im Rahmen der vorliegenden Anmeldung eingesetzt werden können, sei auf einschlägige Lehrbücher verwiesen (siehe beispielsweise K. Sattler, "Thermische Trennverfahren: Grundlagen, Auslegung, Apparate", 3. Auflage, Wiley-VCH, Weinheim 2001).

Bei einer Destillationssäule handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Trenneinsatz) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, auch als Sumpfprodukt bezeichnet, wird in einer Destillationssäule mittels eines Sumpfverdampfers erwärmt, so dass kontinuierlich ein Teil des Sumpfprodukts verdampft und in der Destillationssäule gasförmig aufsteigt. Eine Destillationssäule ist ferner typischerweise mit einer Einrichtung versehen, in die zumindest ein Teil eines sich in einem oberen Bereich der Destillationssäule anreichernden Gasgemischs oder ein entsprechendes Reingas, auch als Kopfprodukt bezeichnet, eingespeist, dort verflüssigt und als flüssiger Rücklauf am Kopf der Destillationssäule aufgegeben wird.

### Vorteile der Erfindung

Für durch Dampfspalten gewonnene Gasgemische sind im Stand der Technik Fraktionierungsverfahren und entsprechende Anlagen in unterschiedlichen Ausgestaltungen bekannt und etabliert. Da sich ein unter Verwendung eines Verfahrens zur oxidativen Kopplung von Methan gebildetes Produkt- bzw. Gasgemisch jedoch insbesondere in seinem Methangehalt deutlich von einem durch Dampfspalten gewonnenen Gasgemisch unterscheidet, können diese jedoch nicht ohne weiteres auf ein derartiges Produkt- bzw. Gasgemisch übertragen werden, bzw. können für Produkt- bzw. Gasgemische, die unter Verwendung eines Verfahrens zur oxidativen Kopplung von Methan gebildet werden, andere Verfahren vorteilhaft sein. Jedoch ähneln die grundsätzlichen Fraktionierungsschritte einander, insbesondere können in beiden Fällen Verfahren zur Demethanisierung bzw. Demethanizer eingesetzt werden.

In einem Verfahren zur Demethanisierung bzw. einem entsprechenden Demethanizer werden aus einem Gasgemisch Verbindungen abgetrennt, die einen niedrigeren Siedepunkt als Ethylen aufweisen, also insbesondere Wasserstoff und Methan. Es verbleibt ein Gasgemisch, das überwiegend oder ausschließlich Kohlenwasserstoffe mit zwei Kohlenstoffatomen sowie ggf. zuvor in dem Gasgemisch enthaltene Kohlenwasserstoffe mit höherem Siedepunkt enthält.

Zur Fraktionierung von durch Dampfspalten gewonnenen Gasgemischen kann beispielsweise eine Demethanizer-First- oder Front-End-Demethanizer-Fraktionierung zum Einsatz kommen. Hierbei steht der Demethanizer an erster Stelle der Fraktionierung. Daher enthält das im Demethanizer bearbeitete Gasgemisch neben den Kohlenwasserstoffen mit zwei Kohlenstoffatomen auch Kohlenwasserstoffe mit drei und mehr Kohlenstoffatomen. In einer Deethanizer-First- oder Frontend-Deethanizer-Fraktionierung werden hingegen Kohlenwasserstoffe mit drei und mehr, in einer Depropanizer-First- oder Frontend- Depropanizer-Fraktionierung Kohlenwasserstoffe mit vier und mehr Kohlenstoffatomen zuvor abgetrennt und gelangen daher nicht mehr in den Demethanizer.

Auch im Rahmen der vorliegenden Erfindung kann grundsätzlich vorab eine Abtrennung von bestimmten schwereren Kohlenwasserstofffraktionen erfolgen; dies ist jedoch aufgrund des geringeren Anteil entsprechender Verbindungen in Gasgemischen, die unter Verwendung eines Verfahrens zur oxidativen Kopplung von Methan gebildet wurden, ggf. nicht zweckmäßig. Vorteilhafterweise wird das Gasgemisch jedoch durch eine Aufbereitung aus einem Produktgemisch des Verfahrens zur oxidativen Kopplung von Methan oder einem Teil hiervon gebildet. Diese Aufbereitung umfasst, da die vorliegende Erfindung eine Tieftemperaturtrennung vorsieht, insbesondere die Abtrennung von Kohlendioxid und die Trocknung des Produktgemischs. Ferner kann eine Entfernung von beispielsweise Kohlenmonoxid und anderer, nicht erwünschter Komponenten vorgenommen werden.

Die vorliegende Erfindung schlägt ein Verfahren zur Demethanisierung eines unter Verwendung eines Verfahrens zur oxidativen Kopplung von Methan gebildeten und Kohlenwasserstoffe mit zwei Kohlenstoffatomen, Wasserstoff und Methan enthaltenden Gasgemischs vor, bei dem unter Verwendung des Gasgemischs eine leichte Fraktion, die zumindest den überwiegenden Anteil des Wasserstoffs und des Methans aus dem Gasgemisch enthält, bereitgestellt wird. Das Gasgemisch kann insbesondere noch geringe Mengen an weiteren Kohlenwasserstoffen, insbesondere mit drei Kohlenstoffatomen, enthalten. Die vorliegende Erfindung bezieht sich damit auf ein Verfahren zur Demethanisierung in einem Demethanizer. Das Kohlenwasserstoffe mit zwei Kohlenstoffatomen, Wasserstoff und Methan enthaltende Gasgemisch kann noch weitere Kohlenwasserstoffe enthalten, beispielsweise Kohlenwasserstoffe mit drei oder Kohlenwasserstoffe mit drei und vier oder Kohlenwasserstoffe mit drei, vier und fünf Kohlenstoffatomen. Diese und höhere Kohlenwasserstoffe können enthalten sein, je nachdem, ob diese in dem Verfahren zur oxidativen Kopplung von Methan gebildet und/oder vorab abgetrennt wurden. Die Kohlenwasserstoffe mit zwei Kohlenstoffatomen umfassen typischerweise Ethylen und Ethan. Acetylen kann, falls vorhanden, in einem entsprechenden Verfahren durch stromauf oder stromab angeordnete Hydrierstufen zu Ethylen umgesetzt worden sein. Das Gasgemisch enthält Methan insbesondere in einem Gehalt von mehr als 40, mehr als 50, mehr als 60, mehr als 70 oder mehr als 80 und bis zu 95, bis zu 90, bis zu 80, bis zu 70 oder bis zu 60 Molprozent. Sämtliche aus den genannten Werten gebildete Teilbereiche können umfasst sein.

Im Rahmen der vorliegenden Erfindung wird das Gasgemisch auf einem ersten Druckniveau einem oder mehreren Abkühlschritten unterworfen. Nach dem oder den Abkühlschritten wird ein (bei einem Abkühlschritt) oder jeweils ein (bei mehreren Abkühlschritten) Kondensat aus dem Gasgemisch abgeschieden. Das oder die entsprechend gebildeten Kondensate werden zusammen mit einem nach dem oder den Abkühlschritten gasförmig verbliebenen Anteil des Gasgemischs auf einem zweiten Druckniveau unterhalb des ersten Druckniveaus einer fraktionierten Destillation unterworfen. Hierzu dient vorteilhafterweise eine Destillationssäule, wie sie grundsätzlich auf dem Gebiet der Demethanisierung bekannt ist. Der nach dem oder den Abkühlschritten gasförmig verbliebene Anteil des Gasgemischs wird dabei erfindungsgemäß arbeitsleistend auf das zweite Druckniveau entspannt, bevor er der fraktionierten Destillation unterworfen wird. Eine "arbeitsleistende Entspannung" erfolgt dabei vorzugsweise in einer Entspannungsmaschine wie einer Expansionsturbine der zuvor erläuterten Art.

Insbesondere durch diese arbeitsleistende Entspannung des gasförmig verbliebenen Anteils des Gasgemischs auf das zweite Druckniveau kann Kälte generiert werden, die es erlaubt, in dem erfindungsgemäßen Verfahren auf weitere Kältemittel, insbesondere C2-Kältemittel, zu verzichten. Das erfindungsgemäße Verfahren kann daher konstruktiv sehr viel einfacher umgesetzt werden, weil keine aufwendigen, zusätzlichen Kältekreisläufe erforderlich sind.

Eine entsprechende Kältegewinnung wäre bei einer Demethanisierung eines Gasgemischs, das unter Verwendung eines Dampfspaltverfahrens erhalten wurden, nicht in dem Umfang wie im Rahmen des erfindungsgemäßen Verfahrens möglich, da solche Gasgemische deutlich mehr Ethylen und ggf. schwerere Kohlenwasserstoffe enthalten. Ethylen und schwerere Kohlenwasserstoffe kondensieren bei einer Entspannung, wie sie im Rahmen der vorliegenden Anmeldung erfolgt. Bei der Kondensation wird dabei latente Wärme abgegeben, es erfolgt jedoch keine weitere Abkühlung. Methan kondensiert im Rahmen des erfindungsgemäßen Verfahrens hingegen nicht oder nur in geringem Umfang und kann daher keine oder kaum latente Wärme abgeben. Bei entsprechend hohen Gehalten an Methan, wie sie zuvor angegeben wurden, reicht daher die Entspannung zur Abkühlung aus.

Mittels der fraktionierten Destillation wird dabei eine gasförmige und überwiegend oder ausschließlich Wasserstoff und Methan enthaltende Kopffraktion gebildet. Die Kopffraktion wird zu einem ersten Anteil abgekühlt, zumindest zum Teil verflüssigt und als Rücklauf in der fraktionierten Destillation eingesetzt. Zu einem zweiten Anteilwird die Kopffraktion einer arbeitsleistenden Entspannung auf ein drittes Druckniveau unterhalb des zweiten Druckniveaus unterworfen, erwärmt und als die leichte Fraktion bereitgestellt. Durch die Entspannung auf das dritte Druckniveau kann weitere Kälte generiert werden, die ebenfalls zur Abkühlung des Gasgemischs und bei der Bildung des oder der Kondensate verwendet werden kann.

Vorteilhafterweise sieht die vorliegende Erfindung vor, dass bei dem oder den Abkühlschritten, denen das Gasgemisch auf dem ersten Druckniveau unterworfen wird, Wärme des Gasgemischs überwiegend oder ausschließlich auf Fluidströme übertragen wird, die aus dem Gasgemisch gebildet werden. Mit anderen Worten wird die Wärmemenge, die dem Gasgemisch bei dem oder den Abkühlschritten insgesamt entzogen wird, überwiegend, insbesondere vollständig, auf aus dem Gasgemisch gebildete Fluidströme übertragen. Ein Wärmetausch mit zusätzlichen Kältemitteln, beispielsweise C2-Kältemitteln, findet vorzugsweise nicht statt. Gleichwohl kann auch die Verwendung zusätzlicher Kältemittel vorgesehen sein.

Zur Gewinnung des Rücklaufs für die fraktionierte Destillation wird der erste Anteil der Kopffraktion vorteilhafterweise einem Wärmetausch mit dem zweiten Anteil der Kopffraktion unterworfen, nachdem dieser der arbeitsleistenden Entspannung unterworfen und damit abgekühlt wurde. Auf diese Weise wird eine besonders effektive Abkühlung ohne den Einsatz zusätzlicher Kältemittel möglich.

Im Rahmen der vorliegenden Erfindung umfassen der oder die Abkühlschritte, denen das Gasgemisch auf dem ersten Druckniveau unterworfen wird, einen Wärmetausch mit dem zweiten Anteil der Kopffraktion, nachdem dieser zuvor dem Wärmetausch mit dem ersten Anteil der Kopffraktion unterworfen wurde. Auf diese Weise kann Restkälte, die nach dem Wärmetausch mit dem ersten Anteil der Kopffraktion noch vorhanden ist, in vorteilhafter Weise zur Abkühlung des Gasgemischs genutzt werden.

Im Rahmen des erfindungsgemäßen Verfahrens wird vorteilhafterwese zur Durchführung des oder der Abkühlschritte, denen das Gasgemisch auf dem ersten Druckniveau unterworfen wird, wenigstens ein erster Wärmetauscher verwendet. Für den Wärmetausch des zweiten Anteils der Kopffraktion mit dem ersten Anteil der Kopffraktion wird vorteilhafterweise ein zweiter Wärmetauscher verwendet. Anstelle eines ersten Wärmetauschers kann auch ein aus mehreren Wärmetauscherblöcken bzw. Wärmetauscherabschnitten bestehender Wärmetauscher oder können mehrere Wärmetauscher eingesetzt werden.

Im Rahmen der vorliegenden Erfindung kommt, wie bereits erwähnt, für die fraktionierte Destillation vorteilhafterweise eine Destillationssäule zum Einsatz, aus der die Kopffraktion an einer Entnahmeposition in deren oberem Bereich abgezogen wird.Vorteilhafterweise ist dabei der zweite Wärmetauscher geodätisch vollständig oberhalb der Entnahmeposition angeordnet. Auf diese Weise kann die Kopffraktion bzw. deren erster Anteil gasförmig in den Wärmetauscher aufsteigen. Nach der Verflüssigung oder Teilverflüssigung kann die dabei gebildete Flüssigkeit ausschließlich durch Schwerkraftwirkung wieder als Rücklauf auf die Destillationssäule abfließen. Auf diese Weise sind keine antreibenden Einrichtungen erforderlich.

Ist es, beispielsweise aus Baubarkeiten, nicht möglich, den zweiten Wärmetauscher geodätisch vollständig oberhalb der Entnahmeposition anzuordnen, und ist daher der der zweite Wärmetauscher geodätisch zumindest teilweise unterhalb oder neben der Entnahmeposition angeordnet, wird vorteilhafterweise der erste Anteil der Kopffraktion dem wenigstens einen zweiten Wärmetauscher mittels eines Gebläses zugeführt. Durch die Gebläsewirkung kann auch die bei der (Teil-)Verflüssigung anfallende Flüssigkeit auf die Destillationssäule zurückgeführt werden.

Die vorliegende Erfindung nutzt vorteilhafterweise zur Abkühlung des Gasgemischs auch das oder die aus dem Gasgemisch gebildeten Kondensate. Vorteilhafterweise umfassen der oder die Abkühlschritte, denen das Gasgemisch auf dem ersten Druckniveau unterworfen wird, daher einen Wärmetausch mit dem oder zumindest einem der aus dem Gasgemisch abgeschieden Kondensate. Die Kondensate können vor dem Wärmetausch insbesondere von dem ersten auf das zweite Druckniveau gedrosselt, also einer Joule-Thomson-Entspannung unterworfen werden, und kühlen hierdurch weiter ab.

In dem Verfahren der vorliegenden Erfindung wird vorteilhafterweise mittels der fraktionierten Destillation eine flüssige, an Wasserstoff und Methan arme oder freie Sumpffraktion gebildet, wobei der oder die Abkühlschritte, denen das Gasgemisch auf dem ersten Druckniveau unterworfen wird, einen Wärmetausch mit der Sumpffraktion umfassen. Der zusätzliche Kältebedarf verringert sich hierdurch weiter.

Wie bereits erwähnt, kann in einem entsprechenden Verfahren der nach dem oder den Abkühlschritten gasförmig verbliebene Anteil des Gasgemischs vor der fraktionierten Destillation arbeitsleistend entspannt und hierdurch weitere Kälte generiert werden.

Vorteilhafterweise werden zur Abkühlung des Gasgemischs auf dem ersten Druckniveau ein erster und ein zweiter Abkühlschritt verwendet, wobei nach dem ersten Abkühlschritt und dem zweiten Abkühlschritt jeweils ein Kondensat aus dem Gasgemisch abgeschieden und der fraktionierten Destillation auf dem zweiten Druckniveau unterworfen wird. Vorteilhafterweise erfolgt dabei der erste Abkühlschritt auf ein Temperaturniveau von -80 bis -90 °C, insbesondere -80 bis -85 °C, beispielsweise ca. -82 °C, und/oder der zweite Abkühlschritt auf ein Temperaturniveau von -90 bis -110 °C, insbesondere -95 bis -100 °C, beispielsweise ca. -98 °C.

Im Rahmen der vorliegenden Erfindung liegt das erste Druckniveau vorteilhafterweise bei 35 bis 40 bar und/oder das zweite Druckniveau bei 20 bis 30 bar, insbesondere 25 bis 27 bar, und/oder das dritte Druckniveau bei 8 bis 12 bar, insbesondere 9 bis 10 bar, beispielsweise ca. 9,7 bar. Die Kopffraktion bei der fraktionierten Destillation wird vorteilhafterweise auf einem Temperaturniveau von -100 bis -110 °C, beispielsweise ca. -105 °C, gebildet. Der Rücklauf zur fraktionierten Destillation wird auf ein Temperaturniveau von -110 bis -120 °C, beispielsweise ca. -115 °C, abgekühlt.

Die vorliegende Erfindung erstreckt sich auch auf einen Demethanizer zur Demethanisierung eines unter Verwendung eines Verfahrens zur oxidativen Kopplung von Methan gebildeten und Kohlenwasserstoffe mit zwei Kohlenstoffatomen, Wasserstoff und Methan und eventuell auch geringere Mengen an Kohlenwasserstoffen mit drei Kohlenstoffatomen enthaltenden Gasgemischs, mit Mitteln, die dafür eingerichtet sind, unter Verwendung des Gasgemischs eine leichte Fraktion, die die zumindest den überwiegenden Anteil des Methans und des Wasserstoffs aus dem Gasgemisch enthält, zu bilden. Erfindungsgemäß sind Mittel vorgesehen, umfassend wenigstens einen ersten Wärmetauscher, die dafür eingerichtet sind, das Gasgemisch auf einem ersten Druckniveau einem oder mehreren Abkühlschritten zu unterwerfen, nach diesem oder diesen ein oder jeweils ein Kondensat aus dem Gasgemisch abzuscheiden, und das oder die Kondensate zusammen mit einem nach dem oder den Abkühlschritten gasförmig verbliebenen Anteil des Gasgemischs auf einem zweiten Druckniveau unterhalb des ersten Druckniveaus einer fraktionierten Destillation zu unterwerfen, wobei eine Entspannungsturbine vorgesehen ist, die dafür eingerichtet ist, den nach dem oder den Abkühlschritten gasförmig verbliebenen Anteil des Gasgemischs arbeitsleistend auf das zweite Druckniveau zu entspannen, bevor er der fraktionierten Destillation unterworfen wird. Eine erfindungsgemäße Anlage ist vorteilhafterweise zur Durchführung eines Verfahrens eingerichtet, wie es zuvor im Detail erläutert wurde. Auf die entsprechenden Merkmale und Vorteile wird daher ausdrücklich verwiesen.

Die Erfindung wird nachfolgend unter Bezugnahme auf die beigefügte Zeichnung näher erläutert, welche eine bevorzugte Ausführungsform der Erfindung zeigt.

### Kurze Beschreibung der Zeichnung

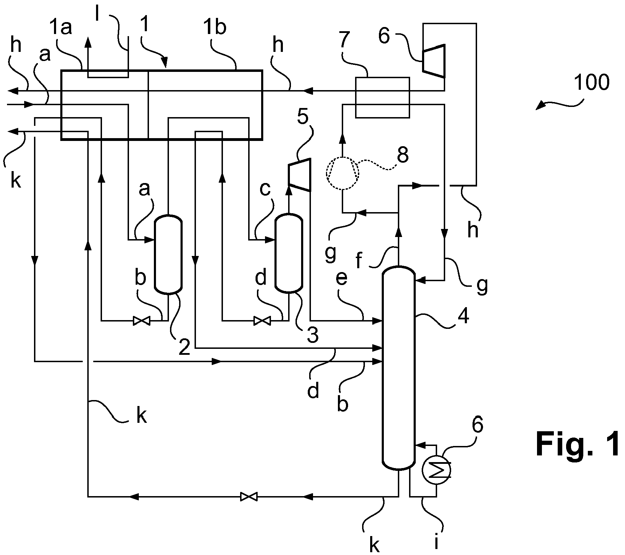

Figur 1 veranschaulicht einen Demethanizer gemäß einer Ausführungsform der Erfindung in Form eines schematischen Prozessflussdiagramrns.

### Ausführliche Beschreibung der Zeichnung

In Figur 1 ist ein Demethanizer gemäß einer besonders bevorzugten Ausführungsform der Erfindung in Form eines schematischen Prozessflussdiagramms veranschaulicht und insgesamt mit 100 bezeichnet. Das in Figur 1 gezeigte Prozessflussdiagramm des Demethanizers 100 beschreibt zugleich ein Verfahren, das unter Verwendung eines entsprechenden Demethanizers 100 durchgeführt werden kann. Wird nachfolgend der Demethanizer 100 beschrieben, gelten die entsprechenden Erläuterungen jederzeit auch für ein derartiges Verfahren.

Dem Demethanizer 100 wird ein Kohlenwasserstoffe mit zwei Kohlenstoffatomen, Wasserstoff und Methan enthaltendes Gasgemisch in Form eines Stroms a zugeführt. Dieser wird zunächst durch einen Wärmetauscher 1 (hier als "erster Wärmetauscher" bezeichnet) geführt und in diesem in der in Figur 1 veranschaulichten Ausführungsform der Erfindung einer zweistufigen Abkühlung unterworfen. Der Wärmetauscher 1 umfasst im dargestellten Beispiel zwei Wärmetauscherblöcke bzw. Wärmetauscherabschnitte 1a und 1b.

Der Strom a wurde dem ersten Wärmetauscher 1 bzw. dessen Wärmetauscherblock 1a bei Umgebungstemperatur oder darunter, insbesondere bei 10 bis 20 °C, beispielsweise bei ca. 15 °C, zugeführt. Nachdem der Strom a durch einen Abschnitt des ersten Wärmetauschers 1 beziehungsweise dessen Wärmetauscherblocks 1a geführt wurde, wird der Strom a dem ersten Wärmetauscher 1 bzw. dessen Wärmetauscherblock 1a auf einem geeigneten Zwischentemperaturniveau, beispielsweise bei ca. -82 °C, entnommen, und in einen Abscheidebehälter 2 überführt. Durch die Abkühlung auf das erläuterte Zwischentemperaturniveau scheidet sich in dem Abscheidebehälter 2 sumpfseitig eine flüssige Fraktion ab, die in Form eines Stroms b abgezogen, über ein nicht gesondert gezeichnetes Ventil gedrosselt, erneut durch einen Abschnitt des ersten Wärmetauschers 1 bzw. dessen Wärmetauscherblocks 1a geführt und dort erwärmt, und schließlich in eine Destillationssäule 4 eingespeist werden kann.

Ein in dem Abscheidebehälter 2 gasförmig verbliebener Anteil des Stroms a wird in Form eines Stroms c erneut in einem Abschnitt des ersten Wärmetauschers 1, nun in dessen Wärmetauscherblock 1 b, abgekühlt, und in einen weiteren Abscheidebehälter 3 überführt. Die Abkühlung erfolgt dabei typischenrveise auf ein Temperaturniveau von ca. -98 °C. Aufgrund dieser Abkühlung scheidet sich auch in dem weiteren Abscheidebehälter 3 eine flüssige Fraktion ab, die in Form des Stroms d abgezogen, gedrosselt, in dem ersten Wärmetauscher 1 bzw. dessen Wärmetauscherblock 1b abgekühlt, und in die Destillationssäule 4 eingespeist werden kann. Ein auch in dem weiteren Abscheidebehälter 3 gasförmig verbliebener Anteil des Stroms a bzw. c kann in Form des Stroms e vorn Kopf des weiteren Abscheidebehälters 3 abgezogen, in einer Entspannungsmaschine 5 arbeitsleistend entspannt und damit abgekühlt und ebenso in die Destillationssäule 4 eingespeist werden.

In der Destillationssäule 4 wird eine gasförmige, überwiegend oder ausschließlich Wasserstoff und Methan enthaltene Kopffraktion gebildet, die vom Kopf der Destillationssäule 4 auf einem Temperaturniveau von typischerweise ca. -105 °C in Form eines Stroms f abgezogen werden kann. Ein Teil des Stroms f, im Rahmen der vorliegenden Anmeldung als "erster Anteil" der Kopffraktion bezeichnet, kann in Form eines Stroms g durch einen Wärmetauscher 7, hier als "zweiter Wärmetauscher" bezeichnet, geführt und dort abgekühlt und zumindest zum Teil verflüssigt werden. Der Strom g kann in verflüssigtem oder teilverflüssigtem Zustand als Rücklauf auf die Destillationssäule 4 zurückgeführt werden, typischerweise auf einem Temperaturniveau von ca. -115 °C. In Figur 1 ist gestrichelt ein Gebläse 8 veranschaulicht, das dafür vorgesehen sein kann, den Strom g durch den Wärmetauscher 7 und den entsprechenden Rücklauf auf die Destillationssäule 4 zurückzuführen, wenn der zweite Wärmetauscher 7 nicht oder nicht vollständig oberhalb der Destillationssäule 7 angeordnet ist.

Zur Bereitstellung von Kälte beziehungsweise zum Entzug von Wärme aus dem Strom g in dem zweiten Wärmetauscher 7 kann ein weiterer Teil des Kopfprodukts der Destillationssäule 4 (hier als "zweiter Anteil" bezeichnet), wie hier in Form des Stroms h veranschaulicht, in einer weiteren Entspannungsmaschine 6 entspannt und dabei abgekühlt werden. Der abgekühlte Strom h wird durch den zweiten Wärmetauscher 7 geführt. Nach dem Passieren des zweiten Wärmetauschers 7 wird der noch kalte Strom h durch den ersten Wärmetauscher 1 bzw. dessen Wärmetauscherblöcke 1b und 1a geführt. Auf diese Weise kann mittels des Stroms h dem Strom a weitere Wärme entzogen werden.

Aus dem Sumpf der Destillationssäule 4 wird ein Sumpfprodukt in Form des Stroms i abgezogen. Dieses kann in einem Sumpfverdampfer 6 verdampft und in die Destillationssäule 4 zurückgespeist werden. Es steigt dort dampfförmig auf und treibt die Destillation. Ein weiterer Teil des Sumpfprodukts der Destillationssäule 4 kann in Form des Stroms k abgezogen, über ein nicht gesondert bezeichnetes Ventil gedrosselt, und ebenfalls in dem ersten Wärmetauscher 1 bzw. dessen Wärmetauscherblock 1a erwärmt werden. Auf diese Weise kann dem Strom a weitere Wärme entzogen werden.

Der Wärmetauscher 1 bzw. dessen Wärmetauscherblock 1a wird ferner von einem Strom I eines C3-Kältemittels durchströmt.

## Patentansprüche

1. Verfahren zur Demethanisierung eines unter Verwendung eines Verfahrens zur oxidativen Kopplung von Methan gebildeten und Kohlenwasserstoffe mit zwei Kohlenstoffatomen, Wasserstoff und Methan enthaltenden Gasgemischs, bei dem unter Verwendung des Gasgemischs eine leichte Fraktion, die zumindest den überwiegenden Anteil des Wasserstoffs und des Methans aus dem Gasgemisch enthält, gebildet wird, **dadurch gekennzeichnet, dass** das Gasgemisch auf einem ersten Druckniveau einem oder mehreren Abkühlschritten unterworfen wird, dass nach dem oder den Abkühlschritten ein oder jeweils ein Kondensat aus dem Gasgemisch abgeschieden wird, dass das oder die Kondensate zusammen mit einem nach dem oder den Abkühlschritten gasförmig verbliebenen Anteil des Gasgemischs auf einem zweiten Druckniveau unterhalb des ersten Druckniveaus einer fraktionierten Destillation unterworfen werden, und dass der nach dem oder den Abkühlschritten gasförmig verbliebene Anteil des Gasgemischs dabei arbeitsleistend auf das zweite Druckniveau entspannt wird, bevor er der fraktionierten Destillation unterworfen wird.

2. Verfahren nach Anspruch 1, bei dem bei der fraktionierten Destillation eine gasförmige und überwiegend oder ausschließlich Wasserstoff und Methan enthaltende Kopffraktion gebildet wird, die zu einem ersten Anteil abgekühlt, zumindest zum Teil verflüssigt und als Rücklauf in der fraktionierten Destillation eingesetzt wird, und die zu einem zweiten Anteil einer arbeitsleistenden Entspannung auf ein drittes Druckniveau unterhalb des zweiten Druckniveaus unterworfen, erwärmt, und als die leichte Fraktion bereitgestellt wird.

3. Verfahren nach Anspruch 1 oder 2, bei dem oder den Abkühlschritten, denen das Gasgemisch auf dem ersten Druckniveau unterworfen wird, Wärme des Gasgemischs auf C3 Kältemittel unu auf Fluidströme übertragen wird, die aus dem Gasgemisch gebildet werden.

4. Verfahren nach Anspruch 2, bei dem der erste Anteil der Kopffraktion einem Wärmetausch mit dem zweiten Anteil der Kopffraktion unterworfen wird, nachdem dieser der arbeitsleistenden Entspannung unterworfen wurde.

5. Verfahren nach Anspruch 4, bei dem der oder die Abkühlschritte, denen das Gasgemisch auf dem ersten Druckniveau unterworfen wird, einen Wärmetausch mit dem zweiten Anteil der Kopffraktion umfassen, nachdem dieser zuvor dem Wärmetausch mit dem ersten Anteil der Kopffraktion unterworfen wurde.

6. Verfahren nach Anspruch 4 oder 5, bei dem zur Durchführung des oder der Abkühlschritte, denen das Gasgemisch auf dem ersten Druckniveau unterworfen wird, wenigstens ein erster Wärmetauscher (1) verwendet wird, und bei dem für den Wärmetausch des zweiten Anteils der Kopffraktion mit dem ersten Anteil der Kopffraktion ein zweiter Wärmetauscher (7) verwendet wird.

7. Verfahren nach Anspruch 6, bei dem für die fraktionierte Destillation eine Destillationssäule (4) verwendet wird, aus der die Kopffraktion an einer Entnahmeposition in deren oberem Bereich abgezogen wird.

8. Verfahren nach Anspruch 7, bei dem der zweite Wärmetauscher (7) vollständig oberhalb der Entnahmeposition angeordnet ist, oder bei dem der zweite Wärmetauscher teilweise unterhalb oder neben der Entnahmeposition angeordnet ist und der erste Anteil der Kopffraktion dem wenigstens einen zweiten Wärmetauscher mittels eines Gebläses (7) zugeführt wird.

9. Verfahren nach einem der vorstehenden Ansprüche, bei dem der oder die Abkühlschritte, denen das Gasgemisch auf dem ersten Druckniveau unterworfen wird, einen Wärmetausch mit dem oder zumindest einem der aus dem Gasgemisch abgeschieden Kondensate umfassen.

10. Verfahren nach einem der vorstehenden Ansprüche, bei dem mittels der fraktionierten Destillation eine flüssige, an Wasserstoff und Methan arme oder freie Sumpffraktion gebildet wird, wobei der oder die Abkühlschritte, denen das Gasgemisch auf dem ersten Druckniveau unterworfen wird, einen Wärmetausch mit dieser Sumpffraktion umfassen.

11. Verfahren nach einem der vorstehenden Ansprüche, bei dem zur Abkühlung des Gasgemisch auf dem ersten Druckniveau ein erster und ein zweiter Abkühlschritt verwendet werden, wobei nach dem ersten Abkühlschritt und dem zweiten Abkühlschritt jeweils ein Kondensat aus dem Gasgemisch abgeschieden und der fraktionierten Destillation auf dem zweiten Druckniveau unterworfen wird.

12. Verfahren nach Anspruch 11, bei dem der erste Abkühlschritt auf ein Temperaturniveau von -80 bis -90 °C und/oder der zweite Abkühlschritt auf ein Temperaturniveau von -90 bis -100 °C erfolgt.

13. Verfahren nach einem der vorstehenden Ansprüche, bei dem das erste Druckniveau bei 30 bis 40 bar und/oder das zweite Druckniveau bei 20 bis 30 bar und/oder das dritte Druckniveau bei 8 bis 12 bar liegt.

14. Verfahren nach einem der vorstehenden Ansprüche, bei dem die Kopffraktion bei der fraktionierten Destillation auf einem Temperaturniveau von -100 bis -110 °C gebildet und/oder der Rücklauf auf einem Temperaturniveau von -110 bis -120 °C in der fraktionierten Destillation verwendet wird.

15. Demethanizer (100) zur Demethanisierung eines unter Verwendung eines Verfahrens zur oxidativen Kopplung von Methan gebildeten und Kohlenwasserstoffe mit zwei Kohlenstoffatomen, Wasserstoff und Methan enthaltenden Gasgemischs, mit Mitteln, die dafür eingerichtet sind, unter Verwendung des Gasgemischs eine leichte Fraktion, die die zumindest den überwiegenden Anteil des Methans und des Wasserstoffs aus dem Gasgemisch enthält, zu bilden, **gekennzeichnet durch** Mittel, umfassend wenigstens einen ersten Wärmetauscher (1), die dafür eingerichtet sind, das Gasgemisch auf einem ersten Druckniveau einem oder mehreren Abkühlschritten zu unterwerfen, nach diesem oder diesen ein oder jeweils ein Kondensat aus dem Gasgemisch abzuscheiden, und das oder die Kondensate zusammen mit einem nach dem oder den Abkühlschritten gasförmig verbliebenen Anteil des Gasgemischs auf einem zweiten Druckniveau unterhalb des ersten Druckniveaus einer fraktionierten Destillation zu unterwerfen, wobei eine Entspannungsturbine (5) vorgesehen ist, die dafür eingerichtet ist, den nach dem oder den Abkühlschritten gasförmig verbliebenen Anteil des Gasgemischs arbeitsleistend auf das zweite Druckniveau zu entspannen, bevor er der fraktionierten Destillation unterworfen wird.
